# EUROPEAN PATENT APPLICATION

(11) **EP 3 741 842 A1**
(43) Date of publication of application: **25.11.2020**
(21) Application number: 19867516.7
(22) Date of filing: 27.09.2019
(51) Int. Cl.: C12N 5/071, A61L 27/38, C12N 5/077

(54) **METHOD FOR CRYOPRESERVING CELLS**

(30) Priority: 27.09.2018 JP 2018182448
(71) Applicant: TERUMO Kabushiki Kaisha, Tokyo 151-0072 (JP)
(72) Inventor: OYAMA, Kenji, Kanagawa 259-0151 (JP); OHASHI, Fumiya, Kanagawa 259-0151 (JP)
(74) Representative: Prüfer & Partner mbB Patentanwälte · Rechtsanwälte
(86) International application number: PCT/JP2019/038195
(87) International publication number: WO 2020/067442

(57) **Abstract**

An object of the present disclosure is to provide a method for cryopreserving cells for transplantation capable of being suitably used for treatment of various diseases, in particular, a heart disease, a method for culturing the cells thawed after cryopreservation, a method for producing a graft containing a sheet-shaped cell culture containing the cells for transplantation, a graft produced by the method, a composition and medical drug containing the graft, and a method for treating a disease using the graft and the like.

The above problems are solved by a method for cryopreserving cells that freezes a cell suspension containing cells for transplantation on a culture substrate, and a method for culturing cells for transplantation that incubates a cell population in a medium containing a cryoprotective agent.

## Description

### Technical Field

The present disclosure relates to a method for cryopreserving cells for transplantation capable of being suitably used for treatment of various diseases, in particular, a heart disease, a method for culturing the cells for transplantation, a method for producing a graft containing the cells for transplantation, a graft produced by the method and containing a sheet-shaped cell culture, a composition and medical drug containing the graft, and a method for treating a disease using the graft and the like.

### Background Art

In recent years, attempts have been made to transplant various cells for repairing damaged tissues and the like. For example, in order to repair cardiac muscular tissues damaged due to ischemic heart diseases such as angina pectoris and myocardial infarction, attempts have been made to use fetal cardiomyocytes, skeletal myoblasts, mesenchymal stem cells, cardiac stem cells, ES cells, iPS cells, and the like (Non Patent Literature 1).

As part of such attempts, a cell structure formed using a scaffold or a sheet-shaped cell culture obtained by forming cells into a sheet shape has been developed (Patent Literature 1 and Non Patent Literature 2).

For applications of the sheet-shaped cell culture for treatment, studies have been conducted on the use of a cultured epidermal sheet for skin damage caused by burns or the like, the use of a sheet-shaped cornea epithelial cell culture for corneal damage, the use of a sheet-shaped oral mucosa cell culture for endoscopic excision of an esophageal cancer and the like. Some of the studies have advanced to clinical application stages.

In order to clinically use the sheet-shaped cell culture, it is required for the sheet-shaped cell culture to be aseptically produced and transferred. However, currently, in order to use the sheet-shaped cell culture and the like, there is no choice but to aseptically produce a sheet-shaped cell culture and the like in a large facility such as a cell processing center (CPC) that is aseptically managed in a medical institution where the sheet-shaped cell culture and the like are used, and the reality is that in a medical institution that does not have such a facility, it is difficult to easily use a sheet-shaped cell culture and the like.

### Citation List

### Patent Literature

Patent Literature 1: JP 2007-528755 A

### Non Patent Literature

Non Patent Literature 1: Haraguchi et al., Stem Cells Transl Med. 2012 Feb;1(2):136-41
Non Patent Literature 2: Sawa et al., Surg Today. 2012 Jan; 42(2) : 181-4

### Summary of Invention

### Technical Problem

An object of the present disclosure is to provide a method for cryopreserving cells for transplantation capable of being suitably used for treatment of various diseases, in particular, a heart disease, a method for culturing the cells thawed after cryopreservation, a method for producing a graft containing a sheet-shaped cell culture containing the cells for transplantation, a graft produced by the method, a composition and medical drug containing the graft, a method for treating a disease using the graft and the like.

### Solution to Problem

As described above, in order to use a graft containing cells for transplantation, such as a sheet-shaped cell culture, it is required for a medical institution to have a facility such as a CPC. During the study of enabling the use of the sheet-shaped cell culture and the like without a facility such as a CPC, the present inventors conceived that, in a case where culture of cryopreserved cells for transplantation and production of a graft can be realized in a movable liquid-tight closed container, a graft such as a sheet-shaped cell culture can be simply used; however, the present inventors faced the problem that some operations such as washing of the cells are very complicated in the liquid-tight closed container. While continuously conducting further intensive studies in order to solve the above problems, the present inventors found that a cell culture with a sufficient medical grade can be obtained even in a case where a sufficient amount of medium is added to a cell suspension obtained by thawing cryopreserved cells and the cells are cultured. As a result of the further studies, the present invention has been completed.

That is, the present invention relates to the following:
[1] A method for cryopreserving cells, including freezing a cell suspension containing cells for transplantation on a culture substrate.
[2] The method according to [1], wherein the cells for transplantation are adhesion cells.
[3] The method according to [1] or [2], wherein the cells for transplantation are myoblasts or cardiomyocytes.
[4] The method according to any one of [1] to [3], wherein the culture substrate has a cell-adhesive surface of cell culture.
[5] The method according to any one of [1] to [4], wherein the culture substrate is covered with a temperature-responsive material.
[6] The method according to any one of [1] to [3], wherein the culture substrate has a culture surface for spheroid formation.
[7] The method according to any one of [1] to [6], wherein the cell suspension contains a cryoprotective agent.
[8] The method according to any one of [1] to [7], wherein the cell suspension contains a 5 to 20% cryoprotective agent.
[9] The method according to any one of [1] to [8], wherein the cell suspension is present on the culture substrate at a density of 5 µL/cm² to 1000 µL/cm² with respect to an area of the culture substrate.
[10] The method according to any one of [1] to [9], wherein the cells for transplantation are present in the cell suspension at a density of 7.5×10⁵ cells/cm² to 3.0×10⁶ cells/cm² with respect to an area of the culture substrate.
[11] A cryopreserved cell frozen on a cell culture substrate.
[12] A method for culturing cells for transplantation, including incubating a cell population in a medium containing a cryoprotective agent.
[13] The method according to [12], wherein the method includes the following steps of:
   (a) obtaining a cell suspension containing a cryoprotective agent by thawing a cell population cryopreserved on a culture substrate;
   (b) diluting the cryoprotective agent by adding a culture medium to the cell suspension obtained in (a); and
   (c) incubating the cell suspension obtained in (b) on the culture substrate.
[14] The method according to [12] or [13], wherein the cell population includes adhesion cells.
[15] The method according to any one of [12] to [14], wherein the cell population includes myoblasts or cardiomyocytes.
[16] The method according to any one of [12] to [15], wherein the cryoprotective agent is DMSO.
[17] The method according to any one of [13] to [16], wherein the culture substrate has a cell-adhesive surface of cell culture.
[18] The method according to any one of [13] to [17], wherein the culture substrate is covered with a temperature-responsive material.
[19] The method according to any one of [13] to [16], wherein the culture substrate has a culture surface for spheroid formation.
[20] The method according to any one of [13] to [19], wherein all of the steps are performed in a liquid-tight closed container.
[21] A method for producing a graft, including the following steps of:
   (A) obtaining a cell suspension containing a cryoprotective agent by thawing a cell population including cells for transplantation, cryopreserved on a culture substrate;
   (B) diluting the cryoprotective agent by adding a culture medium to the cell suspension obtained in (A); and
   (C) performing graft-forming culture on the cell suspension obtained in (B) on the culture substrate.
[22] The method according to [21], wherein the graft is a sheet-shaped cell culture.
[23] The method according to [21] or [22], wherein the cells for transplantation are myoblasts or cardiomyocytes.
[24] The method according to any one of [21] to [23], wherein the cells for transplantation are present in the cell suspension at a density of 7.5×10⁵ cells/cm² to 3.0×10⁶ cells/cm² with respect to an area of the culture substrate.
[25] The method according to any one of [21] to [24], wherein all of the steps are performed in a liquid-tight closed container.
[26] A kit for producing a graft, including:
   a first storage container in which a cell culture substrate and cryopreserved cells frozen on the cell culture substrate are enclosed; and
   a second storage container in which a cell culture medium is enclosed, the second storage container being connectable to the first storage container in a liquid-tight closed connection manner.

### Advantageous Effects of Invention

According to the present invention, cells for transplantation contained in a sheet-shaped cell culture and the like can be simply preserved and used. Therefore, since the graft can be aseptically produced and transferred in the liquid-tight closed container without requiring a complicated procedure or equipment for aseptically producing and transferring the graft, contribution to supply of medical treatment using such a graft can be expected. In addition, since a large facility and the like are not required in the production of such a graft, a cost can be significantly reduced.

### Brief Description of Drawings

Fig. 1 is a graph showing results of thawing and recovering cryopreserved cells. When the cells were suspended in 200 µL or more of a cryoprotective agent (density of 1.0×10⁷ cells/mL or more), there was no significant difference in a recovery rate of cells. There was no significant difference in viability at all concentrations of a suspension.
Fig. 2 shows photographs of sheet-shaped cell cultures obtained by recovering and seeding cryopreserved cells with different densities. In all the cases, there was no problem with sheet-formation.

### Description of Embodiments

Hereinafter, the present disclosure will be described in detail.

All the technical terms and scientific terms used herein have the same meanings understood by those skilled in the art, unless otherwise defined herein. All the patents, applications, published applications, and other publications cited herein are incorporated herein by reference in its entirety. In addition, if any contradiction arises between publications referred to and descriptions herein, the descriptions herein shall take precedence.

In the present disclosure, the term "cells for transplantation" refers to cells to be transplanted in a living body. In the present disclosure, the term "graft" refers to a structure to be transplanted in a living body, and refers to a structure for transplantation containing the cells for transplantation as a constituent component. At least one state in which cells adhere to each other to form a shape as a whole is included in a graft. The so-called suspended state, that is, every single cell exists in a discretely separated state is not included in the "graft" of the present disclosure. In a preferred embodiment, the graft is a structure for transplantation that does not include a structure (for example, a scaffold or the like) other than cells and substances derived from cells. In the present disclosure, the graft is not particularly limited, and examples thereof include a sheet-shaped cell culture, a spheroid, a cell aggregate, a cell suspension, a cell suspension containing fibrin gel, and a cell culture obtained by using nanofibers. The graft is preferably a sheet-shaped cell culture or a spheroid, and more preferably a sheet-shaped cell culture.

In the present disclosure, the term "sheet-shaped cell culture" refers to cells which are connected to each other to form a sheet. In the present disclosure, the term "spheroid" refers to cells which are connected to each other to form an approximately spherical shape. The cells may be connected to each other directly (including via cell components such as adhesion molecules) and/or via an intermediary (interposed) substance. The intermediary substance is not particularly limited as long as it is a substance capable of at least physically (mechanically) connecting cells to each other, and examples thereof can include an extracellular matrix. The intermediary substance is preferably derived from cells, and in particular, derived from cells constituting a sheet-shaped cell culture or a spheroid. The cells are at least physically (mechanically) connected to each other, but may be further functionally connected, for example, chemically or electrically connected to each other. The sheet-shaped cell culture may be composed of one cell layer (monolayer), and may be composed of two or more cell layers (laminate (multilayer), for example, two layers, three layers, four layers, five layers, six layers, and the like). In addition, the sheet-shaped cell culture does not have a clear layer structure of the cells, and may have a three-dimensional structure having a thickness larger than that of one cell. For example, in a vertical cross section of the sheet-shaped cell culture, the cells may not be uniformly aligned in a horizontal direction, but may be present in a state in which a plurality of cells are non-uniformly arranged in a vertical direction (for example, in a mosaic pattern).

The cells (cells for transplantation) constituting a graft such as a sheet-shaped cell culture are not particularly limited as long as cells can form a graft, and examples thereof include adhesion cells (adhesive cells). Examples of the adhesion cells include adhesive somatic cells (for example, cardiomyocytes, fibroblasts, epithelial cells, endothelial cells, liver cells, pancreatic cells, renal cells, adrenal cells, periodontal ligament cells, gingival cells, periosteal cells, skin cells, synoviocytes, chondrocytes, and the like) and stem cells (for example, tissue stem cells such as myoblasts and cardiac stem cells, pluripotent stem cells such as embryonic stem cells and induced pluripotent stem (iPS) cells, mesenchymal stem cells, and the like). The somatic cells may be cells differentiated from stem cells, particularly iPS cells (adhesion cells derived from iPS cells). Non-limiting examples of the cells constituting the graft can include myoblasts (myoblast cells) (for example, skeletal myoblasts and the like), mesenchymal stem cells (for example, cells derived from bone marrow, adipose tissue, peripheral blood, skin, hair root, muscular tissue, endometrium, placenta, umbilical cord blood, and the like), cardiomyocytes, fibroblasts, cardiac stem cells, embryonic stem cells, iPS cells, synoviocytes, chondrocytes, epithelial cells (for example, mouth mucosa epithelial cells, retinal pigment epithelial cells, nasal mucosa epithelial cells, and the like), endothelial cells (for example, vascular endothelial cells and the like), liver cells (for example, hepatic parenchymal cells and the like), pancreatic cells (for example, islet cells and the like), renal cells, adrenal cells, periodontal ligament cells, gingival cells, periosteal cells, and skin cells. Non-limiting examples of the adhesion cells derived from iPS cells can include cardiomyocytes, fibroblasts, epithelial cells, endothelial cells, liver cells, pancreatic cells, renal cells, adrenal cells, periodontal ligament cells, gingival cells, periosteal cells, skin cells, synoviocytes, and chondrocytes derived from iPS cells.

In the present disclosure, the term "myoblasts" refers to precursor cells of striated muscles, and includes skeletal myoblasts and cardiomyoblasts.

In the present disclosure, the term "skeletal myoblasts" refers to myoblasts present in skeletal muscles. The skeletal myoblasts are well known in the technical field, can be produced from skeletal muscles by any known method (for example, a method described in JP 2007-89442 A), and can be commercially available (for example, Lonza, Cat# CC-2580). The skeletal myoblasts can be identified, without limitation, by a marker such as CD56, α7 integrin, myosin heavy chain IIa, myosin heavy chain IIb, myosin heavy chain IId (IIx), MyoD, Myf5, Myf6, myogenin, desmin, or PAX3. In a specific embodiment, the skeletal myoblasts are CD56-positive. In a more specific embodiment, the skeletal myoblasts are CD56-positive and desmin-positive. The skeletal myoblasts may be derived from, without limitation, any organism having a skeletal muscle, for example, mammals such as humans, non-human primates, rodents (mice, rats, hamsters, guinea pigs, and the like), rabbits, dogs, cats, pigs, horses, cows, goats, and sheep. In an embodiment, the skeletal myoblasts are mammalian skeletal myoblasts. In a specific embodiment, the skeletal myoblasts are human skeletal myoblasts.

In the present disclosure, the term "cardiomyoblasts" refers to myoblasts present in a heart muscle. The cardiomyoblasts are well known in the technical field, and can be identified by a marker such as Isl1. The cardiomyoblasts may be derived from, without limitation, any organism having a heart muscle, for example, mammals such as humans, non-human primates, rodents (mice, rats, hamsters, guinea pigs, and the like), rabbits, dogs, cats, pigs, horses, cows, goats, and sheep. In an embodiment, the cardiomyoblasts are mammalian cardiomyoblasts. In a specific embodiment, the cardiomyoblasts are human cardiomyoblasts.

In the present disclosure, the term "cardiomyocytes" refers to cells having characteristics of cardiomyocyte. Examples of the characteristics of cardiomyocyte can include, but are not limited to, expression of cardiomyocyte markers and the presence of autonomous beating. Non-limiting examples of a cardiomyocyte marker can include cardiac troponin T (c-TNT), CD172a (also known as SIRPA or SHPS-1), KDR (also known as CD309, FLK1, or VEGFR2), PDGFRA, EMILIN2, and VCAM. Preferred examples of cardiomyocytes can include cardiomyocytes derived from iPS cells.

The cells constituting the graft can be derived from any organism which can be treated by the graft. Examples of the organism include, but are not limited to, humans, non-human primates, dogs, cats, pigs, horses, goats, sheep, rodents (mice, rats, hamsters, guinea pigs, and the like), and rabbits. In addition, the number of types of cells constituting the graft is not particularly limited, but the graft may be composed of only one type of cell, or 2 or more types of cells may be used for the graft. In a case where the number of types of cells constituting the graft is 2 or more, a content percentage (purity) of cell whose number is the largest is 50% or more, preferably 60% or more, more preferably 70% or more, and still more preferably 75% or more, when the formation of the graft is finished.

The sheet-shaped cell culture of the present disclosure preferably does not include a scaffold (support). A scaffold may be used in the technical field to maintain physical integrity of a sheet-shaped cell culture by allowing adhesion of cells onto a surface and/or an internal portion of the scaffold. For example, a film formed of polyvinylidene difluoride (PVDF) and the like are known, but the sheet-shaped cell culture of the present disclosure can maintain its physical integrity without such a scaffold. In addition, the sheet-shaped cell culture of the present disclosure is preferably formed of only substances (an extracellular matrix and the like) derived from cells constituting a sheet-shaped cell culture, and does not contain substances other than these substances.

The cells may be heterologous cells (xenogeneic cells) or homologous cells (allogeneic cells). Here, the term "heterologous cells" refers to cells derived from an organism whose species is different from that of a recipient, in the case where the sheet-shaped cell culture is used for transplantation. For example, in a case where the recipient is a human, cells derived from a monkey or a pig correspond to the heterologous cells. In addition, the term "homologous cells" refers to cells derived from an organism whose species is the same as that of a recipient. For example, in a case where the recipient is a human, human cells correspond to the homologous cells. The homologous cells include self-derived cells (also referred to as self-cells or autologous cells), that is, cells derived from the recipient, and homologous non-autologous cells (also referred to as allogeneic cells). Since the self-derived cells do not cause rejection when transplanted, the self-derived cells are preferable in the present disclosure. However, the heterologous cells or the homologous non-autologous cells can also be used. In the case where heterologous cells or homologous non-autologous cells are used, an immunosuppressive treatment may be required to suppress rejection. Note that, herein, cells other than the self-derived cells, that is, the heterologous cells and the homologous non-autologous cells may be collectively referred to as non- self-derived cells. In an embodiment of the present disclosure, the cells are autologous cells or allogeneic cells. In an embodiment of the present disclosure, the cells are autologous cells (including autologous cells derived from autologous iPS cells and autologous differentiation-induced cells obtained by differentiating and inducing autologous iPS cells). In another embodiment of the present disclosure, the cells are allogeneic cells (including allogeneic cells derived from allogeneic iPS cells and allogeneic differentiation-induced cells obtained by differentiating and inducing allogeneic iPS cells).

A sheet-shaped cell culture can be produced by any known method (for example, see Patent Literature 1, Non Patent Literature 2, JP 2010-081829 A, and JP 2011-110368 A). Typically, a method for producing a sheet-shaped cell culture includes: seeding cells on a culture substrate; performing sheet-formation on the seeded cells; and detaching the formed sheet-shaped cell culture from the culture substrate, but the present invention is not limited thereto. A step of freezing the cells and a step of thawing the cells may be performed before the step of seeding the cells on the culture substrate. Further, a step of washing the cells may be performed after the step of thawing the cells. Each of the steps can be performed by any known method suitable for producing a sheet-shaped cell culture. A production method of the present disclosure may include a step of producing a sheet-shaped cell culture. In this case, the step of producing the sheet-shaped cell culture may include 1 or 2 or more steps according to the method for producing a sheet-shaped cell culture, as a sub-step. In an embodiment, a step of proliferating the cells after the step of thawing the cells and before the step of seeding the cells on the culture substrate is not included.

The spheroid can be produced by any known method (for example, see JP 5523830 B2). Typically, a method for producing a spheroid includes: seeding cells on a culture substrate having a concave portion; spheroidizing the seeded cells; and obtaining the formed spheroid from the culture substrate, but the present invention is not limited thereto. For example, examples of the spheroid can include a graft obtained by forming cardiomyocytes derived from pluripotent stem cells into 1000 spherical forms having a diameter of 0.2 mm.

The culture substrate is not particularly limited as long as cells can form a cell culture on the culture substrate, and examples thereof can include a container formed of various materials and/or formed in various shapes and a container having a solid or semi-solid surface. A structure and material of the container preferably do not allow permeation of a liquid such as a culture solution. Examples of the material can include, but are not limited to, polyethylene, polypropylene, Teflon (registered trademark), polyethylene terephthalate, polymethyl methacrylate, nylon 6,6, polyvinyl alcohol, cellulose, silicon, polystyrene, glass, polyacrylamide, polydimethylacrylamide, and a metal (for example, iron, stainless steel, aluminum, copper, or brass). In addition, the container preferably has at least one flat surface. Examples of the container can include, but are not limited to, a culture container having a bottom surface including a culture substrate capable of forming a cell culture and a liquid impermeable side surface. Specific examples of the culture container can include, but are not limited to, a cell culture dish and a cell culture bottle. The bottom surface of the container may be transparent or opaque. When the bottom surface of the container is transparent, cells can be observed, counted, and the like, from a back side of the container. In addition, the container may have a solid or semi-solid surface therein. Examples of the solid surface can include a plate or container formed of various materials described above. Examples of the material for the semi-solid surface can include gel and a soft polymer matrix. The culture substrate may be produced using the above materials, or a commercially available culture substrate may be used.

A preferred culture substrate is not limited, and examples thereof can include a substrate having an adhesive surface suitable for the formation of the sheet-shaped cell culture, and a substrate having a low adhesive surface, and/or a substrate having a uniform well-like structure that are suitable for the formation of the spheroid. Specifically, in the case of the formation of the sheet-shaped cell culture, examples of the substrate can include a substrate having a surface coated with a hydrophilic compound such as polystyrene, collagen gel, or a hydrophilic polymer which is subjected to corona discharge treatment, and a substrate having a surface coated with an extracellular matrix such as collagen, fibronectin, laminin, vitronectin, proteoglycan, or glycosaminoglycan, or a cell adhesion factor such as cadherin family, selectin family, or integrin family. In addition, the substrate is commercially available (for example, Corning (registered trademark) TC-Treated Culture Dish, Corning, Inc., or the like). In addition, in the case of the formation of the spheroid, examples of the substrate can include a substrate having a surface coated with a non-cell adhesive compound such as hydrogel such as soft agar, temperature responsive gel (commercial name: Mebiol gel) obtained by crosslinking of poly(N-isopropylacrylamide) (PIPAAm) with polyethylene glycol (PEG), polyhydroxyethyl methacrylate (polyHEMA), a 2-methacryloyloxyethyl phosphorischoline (MPC) polymer, and/or a substrate having a surface having a uniform rugged structure. The substrate is also commercially available (for example, EZSPHERE (registered trademark) or the like). The culture substrate may be entirely or partially transparent or opaque.

The surface of the culture substrate may be covered with a material whose physical properties are changed in response to a stimulus, for example, temperature or light. Examples of the material can include, but are not limited to, known materials such as a temperature-responsive material composed of a homopolymer or a copolymer of a (meth)acrylamide compound, an N-alkyl-substituted (meth)acrylamide derivative (for example, N-ethylacrylamide, N-n-propylacrylamide, N-n-propylmethacrylamide, N-isopropylacrylamide, N-isopropylmethacrylamide, N-cyclopropylacrylamide, N-cyclopropylmethacrylamide, N-ethoxyethylacrylamide, N-ethoxyethylmethacrylamide, N-tetrahydrofurfurylacrylamide, N-tetrahydrofurfurylmethacrylamide, or the like), an N,N-dialkyl-substituted (meth)acrylamide derivative (for example, N,N-dimethyl(meth)acrylamide, N,N-ethylmethylacrylamide, N,N-diethylacrylamide, or the like), a (meth)acrylamide derivative having a cyclic group (for example, 1-(1-oxo-2-propenyl)-pyrrolidine, 1-(1-oxo-2-propenyl)-piperidine, 4-(1-oxo-2-propenyl)-morpholine, 1-(1-oxo-2-methyl-2-propenyl)-pyrrolidine, 1-(1-oxo-2-methyl-2-propenyl)-piperidine, 4-(1-oxo-2-methyl-2-propenyl)-morpholine, or the like), or a vinyl ether derivative (for example, methyl vinyl ether), a light-absorbing polymer having an azobenzene group, and a light-responsive material such as a copolymer of a vinyl derivative of triphenylmethane leuco hydroxide and an acrylamide-based monomer and N-isopropylacrylamide gel containing spirobenzopyran (for example, see JP H2-211865 A and JP 2003-33177 A). By providing a certain stimulus to these materials, the physical properties such as hydrophilicity and hydrophobicity can be changed, and detachment of a cell culture adhering to the materials can be promoted. Culture dishes covered with a temperature-responsive material are commercially available (for example, UpCell (registered trademark) of CellSeed Inc.), and the culture dishes can be used for the production method of the present disclosure.

The culture substrate may have various shapes. In addition, an area thereof is not particularly limited, and may be, for example, about 1 cm² to about 200 cm², about 2 cm² to about 100 cm², or about 3 cm² to about 50 cm². For example, examples of the culture substrate can include a circular culture dish having a diameter of 10 cm. In this case, an area thereof is 56.7 cm². A culture surface may be flat or may have a rugged structure. In a case where the culture surface has an uneven structure, a uniformly rugged structure is preferable.

The culture substrate may be coated (covered or coated) with serum. By using the culture substrate coated with serum, a sheet-shaped cell culture having a higher density can be formed. The expression "coated with serum" refers to a state in which a serum component adheres to a surface of the culture substrate. The state is not limited and can be obtained, for example, by treating the culture substrate with serum. The treatment with serum includes contact of the serum with the culture substrate, and if necessary, incubation for a predetermined period.

As the serum, heterologous serum and/or homologous serum can be used. The heterologous serum refers to serum derived from an organism whose species is different from that of a recipient, in the case where the sheet-shaped cell culture is used for transplantation. For example, when the recipient is a human, serum derived from a cow or a horse, such as fetal bovine serum (FBS, FCS), calf serum (CS), or horse serum (HS) corresponds to the heterologous serum. In addition, the term "homologous serum" refers to serum derived from an organism whose species is the same as that of a recipient. For example, in a case where the recipient is a human, human serum corresponds to the homologous serum. The homologous serum includes self-serum (also referred to as autologous serum), that is, serum derived from the recipient and homologous allogeneic serum derived from individuals of the same species other than the recipient. Note that, herein, serum other than the self-serum, that is, the heterologous serum and the homologous allogeneic serum may be collectively referred to as non-self-serum.

The serum for coating the culture substrate is commercially available, or can be prepared by a common method from a blood collected from a desired organism. Specifically, as an example, a method in which collected blood is coagulated by leaving the blood at room temperature for about 20 to about 60 minutes and centrifuged at about 1000×g to about 1200×g to collect a supernatant may be used.

When serum is incubated on the culture substrate, undiluted serum or diluted serum may be used. The dilution can be performed, without limitation, with any medium such as water, physiological saline, various buffers (for example, PBS, HBSS, and the like) and various liquid media (for example, DMEM, MEM, F12, DMEM/F12, DME, RPMI1640, MCDB (MCDB102, 104, 107, 120, 131, 153, 199, and the like), L15, SkBM, RITC80-7, and the like). A dilution concentration is not particularly limited as long as the serum component can adhere onto the culture substrate. For example, the dilution concentration is about 0.5% to about 100% (v/v), preferably about 1% to about 60% (v/v), and more preferably about 5% to about 40% (v/v).

Incubation time is also not particularly limited as long as the serum component can adhere onto the culture substrate. For example, the incubation time is about 1 hour to about 72 hours, preferably about 2 hours to about 48 hours, more preferably about 2 hours to about 24 hours, and still more preferably 2 hours to about 12 hours. An incubation temperature is also not particularly limited as long as the serum component can adhere onto the culture substrate. For example, the incubation temperature is about 0°C to about 60°C, preferably about 4°C to about 45°C, and more preferably room temperature to about 40°C.

The serum may be discarded after the incubation. As a method for discarding serum, general methods for discarding a liquid such as suction with a pipette and decantation can be used. In a preferred embodiment of the present disclosure, the culture substrate may be washed with a serum-free washing solution after the serum is discarded. The serum-free washing solution is not particularly limited as long as it is a liquid medium which does not contain serum and does not adversely affect the serum component adhering to the culture substrate. Examples thereof can include, but are not limited to, water, physiological saline, various buffers (for example, PBS, HBSS, and the like) and various liquid media (for example, DMEM, MEM, F12, DMEM/F12, DME, RPMI1640, MCDB (MCDB102, 104, 107, 120, 131, 153, 199, and the like), L15, SkBM, RITC80-7, and the like). As a washing method, general methods for washing a culture substrate such as a method in which a serum-free washing solution is added to the culture substrate, and the solution is stirred for a predetermined time (for example, about 5 seconds to about 60 seconds) and then discarded can be used without limitation.

In the present disclosure, the culture substrate may be coated with a growth factor. Here, the term "growth factor" refers to an arbitrary substance which promotes proliferation of cells as compared to a case without the substance. Examples thereof include an epithelial growth factor (EGF), a vascular endothelial growth factor (VEGF), and a fibroblast growth factor (FGF). A method for coating a culture substrate with a growth factor, a discarding method, and a washing method are basically the same as those for serum, except that the dilution concentration during the incubation is, for example, about 0.0001 µg/mL to about 1 µg/mL, preferably about 0.0005 µg/mL to about 0.05 µg/mL, and more preferably about 0.001 µg/mL to about 0.01 µg/mL.

In the present disclosure, the culture substrate may be coated with a steroid. Here, the term "steroid" refers to, among compounds having a steroid nucleus, a compound which may have adverse effects such as adrenocortical insufficiency and Cushing's syndrome on a living body. Examples of the compound can include, but are not limited to, cortisol, prednisolone, triamcinolone, dexamethasone, and betamethasone. A method for coating a culture substrate with a steroid, a discarding method, and a washing method are basically the same as those for serum, except that the dilution concentration during the incubation is, for example, about 0.1 µg/mL to about 100 µg/mL, preferably about 0.4 µg/mL to about 40 µg/mL, and more preferably about 1 µg/mL to about 10 µg/mL.

The culture substrate may be coated with any one of serum, a growth factor, and a steroid or may be coated with any combination thereof, that is, combination of: serum and a growth factor; serum and a steroid; serum, a growth factor, and a steroid; or a growth factor and a steroid. In a case where the culture substrate is coated with a plurality of components, these components may be mixed and simultaneously coated, or may be coated according to separate steps.

### <1> Cryopreservation Method of Present Disclosure

An aspect of the present disclosure relates to a method for cryopreserving cells for transplantation. The cryopreservation method of the present disclosure includes freezing a cell suspension containing cells for transplantation on a culture substrate. In the related art, when cells are cryopreserved, the cryopreservation is performed by enclosing a cell suspension in a cryopreservation container such as a cryopreservation vial and freezing the cell suspension. However, in the method of the present disclosure, the cells for transplantation are cryopreserved by freezing a cell suspension directly on a culture substrate rather than in a cryopreservation container. Therefore, cryopreserved cells prepared by the cryopreservation method of the present disclosure, and cryopreserved cells typically frozen on a cell culture substrate are also included in the invention of the present disclosure.

As a method for freezing a cell suspension, any method known in the technical field may be used, and for example, a method in which a cell suspension is soaked in liquid nitrogen and the soaked cell suspension is cooled in a program freezer may be used. The time it takes for the cell suspension to freeze is not particularly limited, but the time when the cells are less damaged or a state of the cells is not changed is preferable. For example, when adhesion cells are cryopreserved on a cell adhesive culture substrate, the adhesion cells are preferably frozen before the adhesion cells adhere to the culture substrate. Therefore, the time from the start to the end of the freezing is not particularly limited. The freezing may be performed, for example, within about 24 hours, within about 12 hours, within about 10 hours, within about 8 hours, within about 6 hours, within about 5 hours, within about 4 hours, within about 3 hours, within about 2 hours, within about 1 hour, within about 30 minutes, or the like.

The cells cryopreserved by the method of the present disclosure are not particularly limited as long as they are cells used for transplantation, and any cells for transplantation can be used. The cells for transplantation are as described above. In a preferred embodiment, the cells for transplantation are adhesion cells. In a more preferred embodiment, the cells for transplantation are myoblasts or cardiomyocytes.

A medium suspending the cryopreserved cells may be any medium as long as it is used for cryopreservation of the cells in the technical field. Examples thereof can include, but are not limited to, a basal medium such as DMEM, MEM, F12, DME, RPMI1640, MCDB (MCDB102, 104, 107, 120, 131, 153, 199, or the like), L15, SkBM, RITC80-7, or DMEM/F12, and a buffer solution such as a Hank's balanced salt solution (HBSS), an Earle's balanced salt solution (EBSS), or a phosphate buffer solution (PBS).

The cells for transplantation cryopreserved by the method of the present disclosure are thawed and then used for culture as they are. Therefore, the culture substrate used in the method of the present disclosure is not particularly limited as long as it can withstand the freezing treatment and can be suitably used for culture of cells for transplantation. For example, when the cells for transplantation are adhesion cells, the culture substrate preferably has a cell-adhesive surface of cell culture. In addition, the culture substrate can also be changed by the use of the cells for transplantation. For example, for the purpose of producing a sheet-shaped cell culture from the cells for transplantation, the culture substrate has a cell-adhesive surface of cell culture, preferably, a culture surface coated with a temperature-responsive material. For the purpose of forming a spheroid, the culture substrate preferably has a culture surface for spheroid formation, for example, a low adhesive surface and/or a culture surface having a uniform well-shaped structure. Those skilled in the art can select a culture substrate having appropriate properties by taking into consideration cryopreserved cells for transplantation, the use thereof, and the like.

In a case where cells for transplantation are cryopreserved by the method of the present disclosure, a support, a scaffold, or the like (hereinafter, collectively referred to as a "support or the like") may be cryopreserved together. Therefore, the support or the like may be included in the cell suspension for the cells for transplantation. Any support or the like may be used as long as it is advantageous in terms of forming a graft. It is preferable that a material for the support or the like does not have an adverse effect when transplanted in a living body. It is more preferable that the material is biodegradable. Examples of the material can include polylactic acid and fibrin gel. Therefore, examples of the support or the like can include a film or support formed of a biodegradable polymer such as polylactic acid, polyglycolic acid, or polycaprolactone, and a support formed of a living body-derived component such as fibrin gel.

The cell suspension cryopreserved on the culture substrate preferably contains a cryoprotective agent in order to prevent freezing damage. As the cryoprotective agent that can be used in the cryopreservation method of the present disclosure, any cryoprotective agent generally used in the technical field can be used as long as it does not contain impurities such as serum in a production process in consideration of preservation of cells for transplantation. Examples thereof can include, but are not limited to, polyhydric alcohol such as dimethyl sulfoxide (DMSO) or glycerol, sugars such as trehalose, and polyamino acid such as polylysine. A content of the cryoprotective agent is not limited as long as the cells can be protected from damage during freezing and are not adversely affected. The content of the cryoprotective agent may be about 1 to 20%, about 5 to 15%, about 7 to 12%, or the like.

The amount of cell suspension to be cryopreserved is not particularly limited as long as the cell suspension can be suitably frozen on the culture substrate. When the amount of the cell suspension is too large, it takes a long time to freeze the cell suspension and freezing damage easily occurs. Therefore, it is not preferable to use more than the appropriate amount of the cell suspension. The amount of the cell suspension is preferably about 5 to 1000 µL/cm², and more preferably about 10 to 300 µL/cm², with respect to an area of the culture substrate.

The amount of cells to be cryopreserved can be changed depending on a size of the culture substrate and can be determined in consideration of the purpose of culture after thawing. In a case where the cells are thawed and then used for proliferation culture, the amount of the cells in the cell suspension may be, for example, about 7.5×10⁵ cells/cm² to 3.0×10⁶ cells/cm² with respect to the area of the culture substrate.

In an embodiment, the cryopreserved cells are thawed and then used for graft-forming culture. In the present disclosure, the term "graft-forming culture" refers to culture for forming cells in culture as a graft. In particular, a case where the graft is a sheet-shaped cell culture is referred to as "sheet-forming incubation". The cell-sheet formation can be performed by any known method and condition. Non-limiting examples of the method are described in JP 2010-081829 A, JP 2010-226991 A, JP 2011-110368 A, JP 2011-172925 A, WO 2014/185517 A, and the like. It is considered that graft formation of cells (for example, sheet formation) is achieved by cells adhering to each other via an intercellular adhesion mechanism such as an adhesion molecule or an extracellular matrix. Therefore, the graft-forming culture can be achieved by culturing the cells under a condition in which intercellular adhesion is formed. Any condition may be set as long as the intercellular adhesion can be formed. However, in general, the intercellular adhesion can be formed under the same condition as a general cell culture condition. Examples of the condition can include a condition in which culture is performed at about 37°C and 5% CO₂. In addition, the culture can be performed under a normal pressure (under atmospheric pressure or non-pressure). In the graft-forming culture (sheet-forming incubation), it is not necessary for cells to proliferate, as long as the intercellular adhesion is formed. In a preferred embodiment, the graft-forming culture (sheet-forming incubation) is performed without proliferation of cells.

In a case where the cryopreserved cells are thawed and then used for the graft-forming culture, the amount of the cryopreserved cells (that is, the cells for transplantation) may be, but is not limited to, for example about 5.0×10⁵ cells/cm² to about 1.0×10⁷ cells/cm², about 5.0×10⁵ cells/cm² to about 5.0×10⁶ cells/cm², about 5.0×10⁵ cells/cm² to about 3.0×10⁶ cells/cm², about 1.0×10⁶ cells/cm² to about 1.0×10⁷ cells/cm², about 1.0×10⁶ cells/cm² to about 5.0×10⁶ cells/cm², about 1.0×10⁶ cells/cm² to about 3.0×10⁶ cells/cm², about 1.5×10⁶ cells/cm² to about 1.0×10⁷ cells/cm², about 1.5×10⁶ cells/cm² to about 5.0×10⁶ cells/cm², about 1.5×10⁶ cells/cm² to about 3.0×10⁶ cells/cm², about 2.0×10⁶ cells/cm² to about 1.0×10⁷ cells/cm², about 2.0×10⁶ cells/cm² to about 5.0×10⁶ cells/cm², or about 2.0×10⁶ cells/cm² to about 3.0×10⁶ cells/cm², with respect to the area of the culture surface of the culture substrate. In a preferred embodiment, the amount of the cryopreserved cells is about 7.5×10⁵ cells/cm² to about 3.0×10⁶ cells/cm², and in another preferred embodiment, the amount of the cryopreserved cells is about 1.76×10⁶ cells/cm² to about 2.33×10⁶ cells/cm².

A volume of the cell suspension is not particularly limited as long as the above-described amount of the cells can be suspended. However, when the volume of the cell suspension is too large, the culture substrate is also required to be relatively large. Therefore, it is preferable that the volume of the cell suspension is not too large. Non-limiting examples of the volume of the cell suspension may be about 10 mL, about 11 mL, about 12 mL, about 13 mL, about 14 mL, about 15 mL, about 16 mL, about 17 mL, about 18 mL, about 19 mL, about 20 mL, about 21 mL, about 22 mL, about 23 mL, about 24 mL, about 25 mL, about 26 mL, about 27 mL, about 28 mL, about 29 mL, and about 30 mL.

A capacity of the culture substrate cryopreserving cells is not particularly limited as long as it can contain cells. It is preferable that the capacity of the culture substrate is larger than that of the cell suspension in view of adding a medium later. In an embodiment, the capacity of the culture substrate may be about 5 times, about 6 times, about 7 times, about 8 times, about 9 times, or about 10 times larger than that of the cell suspension.

The cryopreserved cells of the present disclosure may be thawed and then used for culture on the culture substrate as they are. In a case where the cells are thawed and then used for culture as they are, a medium used for culture is added to the obtained thawed cell suspension to be used for the culture. Details of the culture are as described in a culture method to be described below. In a preferred embodiment, when performing the graft-forming culture, a step of thawing the frozen cells and then washing the cells (replacing the cell culture solution) before performing graft-forming culture is not included.

### <2> Culture Method of Present Disclosure

An aspect of the present disclosure relates to a method for culturing cells for transplantation characterized in that cells are incubated in a medium containing a cryoprotective agent. In the culture method of the present disclosure, the cryoprotective agent and the cells for transplantation are as described in <1> above. The culture method can be typically performed after thawing the cryopreserved cells by the cryopreservation method described in <1> above.

Hereinafter, the present invention will be described with reference to the case where cryopreserved cells are cultured by the cryopreservation method of <1> by way of example.

In a typical embodiment, the culture method of the present disclosure includes the following steps of:
(a) obtaining a cell suspension containing a cryoprotective agent by thawing a cell population cryopreserved on a culture substrate;
(b) diluting the cryoprotective agent by adding a culture medium to the cell suspension obtained in (a); and
(c) incubating the cell suspension obtained in (b) on the culture substrate.

In the step (a), the cells cryopreserved on the culture substrate are thawed. Any method known in the technical field may be used for the thawing as long as it does not excessively damage the cryopreserved cells. For example, the thawing can be performed by methods such as water bath, hat water bath, natural thawing, and quick thawing by adding warmed medium. The cell suspension dispersed in a cryoprotective agent can be obtained by the step (a).

In the step (b), the culture medium is added to the cell suspension obtained in the step (a). By doing so, the cryoprotective agent contained in the cell suspension obtained in (a) is diluted. The culture medium is not particularly limited as long as it is generally used in the technical field, and for example, a medium based on physiological saline, various buffers (for example, PBS, HBSS, and the like) and various basal media for cell culture may be used. Examples of the basal medium can include, but are not limited to, DMEM, MEM, F12, DME, RPMI1640, MCDB (MCDB102, 104, 107, 120, 131, 153, 199, and the like), L15, SkBM, RITC80-7, and DMEM/F12. Most of the basal media are commercially available, and a composition thereof is known. A standard composition of the basal medium may be used as it is (for example, as it is commercially available), and a composition of the basal medium may be appropriately changed depending on a type of cell or a cell condition. Therefore, the basal medium used in the present invention is not limited to a known composition, and examples thereof include any basal medium in which one or two or more components are added, removed, increased, or decreased. A graft-forming medium may contain additives such as general serum (for example, bovine serum such as fetal bovine serum, horse serum, human serum, or the like), and various growth factors (for example, FGF, EGF, VEGF, HGF, and the like). However, in a case where a sheet-shaped cell culture is produced under a xeno-free condition, it is particularly preferable that heterologous serum such as bovine serum or horse serum is not contained. A feature of the present disclosure is that the graft-forming culture is performed by using a graft-forming medium containing a cell adhesive component. Accordingly, the graft-forming medium can implement graft-formation with high quality even in a case where the graft-forming medium does not contain serum. Therefore, in a preferred embodiment, the graft-forming medium does not contain serum.

The amount of medium to be added is not particularly limited as long as the cryoprotective agent can be diluted to a concentration sufficient to culture the cryopreserved cells. For example, in a case where about 7.5×10⁵ cells/cm² to 3.0×10⁶ cells/cm² of cells are cryopreserved in a circular culture dish having a diameter of 10 cm, the amount of medium to be added may be about 5 to 30 mL, and in a preferred embodiment, the amount of medium to be added may be 5 to 10 mL. A dilution ratio of the cryopreservation solution may be 2 times to 100 times, and in a preferred embodiment, the dilution ratio of the cryoprotective agent may be 5 times to 15 times.

In the step (c), the cell suspension to which the culture medium is added in (b) is incubated on the culture substrate. The culture in the culture method of the present disclosure includes not only general culture for proliferating cells, but also culture in which the number of cells is not substantially changed, such as graft-forming culture (typically sheet-forming incubation) for forming a graft from a cell population.

The culture method of the present disclosure can be used for any cell population. In a preferred embodiment, a cell population to be cultured includes cells for transplantation. The cells for transplantation used in the culture method of the present disclosure are as described above. Therefore, in a preferred embodiment, the cell population includes adhesion cells, and in a more preferred embodiment, the cell population includes myoblasts or cardiomyocytes.

In the culture method of the present disclosure, a medium (cell suspension) containing a cryoprotective agent may be added to a culture medium, or a culture medium may be added to a medium containing a cryoprotective agent. Typically, as described above, the culture medium is added to a medium (cell suspension) containing a cryoprotective agent. The cryoprotective agent is as described in <1> above. In a preferred embodiment, the cryoprotective agent is DMSO.

In the culture method of the present disclosure, the cell population cryopreserved by the method of <1> is typically used for culture as it is. Therefore, the culture substrate used in the culture method of the present disclosure is the same as described in <1> above. That is, in a preferred embodiment, the culture substrate used in the culture method of the present disclosure has a cell-adhesive surface of cell culture, and in a more preferred embodiment, the culture substrate has a culture surface covered with a temperature-responsive material. In addition, in another preferred embodiment, the culture substrate has a culture surface for spheroid formation. Such a culture substrate is known in the technical field as described above and is commercially available.

The incubation as described above may be incubation in which the number of cells is not substantially changed. Such incubation is typical graft-forming culture. Accordingly, in a particularly preferred embodiment of the present disclosure, the step (c) is a step of performing graft-forming culture. When the step (c) is a step of performing graft-forming culture, the resulting cell culture is a graft. Therefore, a preferred embodiment of the present disclosure relates to a method for producing a graft, the method including the following steps of:
(A) obtaining a cell suspension containing a cryoprotective agent by thawing a cell population including cells for transplantation, cryopreserved on a culture substrate;
(B) adding a culture medium to the cell suspension obtained in (A); and
(C) performing graft-forming culture on the cell suspension obtained in (B) on the culture substrate.

In a method for producing a graft of the present disclosure, the steps (A) and (B) are as described above in the steps (a) and (b) in the culture method.

The graft-forming culture in the step (C) is typical sheet-forming incubation. Details of the sheet-forming incubation are as described above. Therefore, in a preferred embodiment of the method for producing a graft of the present disclosure, the graft is a sheet-shaped cell culture. The graft-forming culture in the step (C) may be culture for forming a spheroid. After the graft-forming culture in the step (C), a step of diluting the cryoprotective agent in the graft-forming culture or a step of performing washing to remove the cryoprotective agent from the graft-forming culture may be further included.

In the method for producing a graft, the cells for transplantation are not particularly limited as long as a graft can be formed, and are typically adhesion cells. In a preferred embodiment, the cells for transplantation are myoblasts or cardiomyocytes. The cardiomyocytes may be cardiomyocytes derived from iPS cells.

In culture for forming a graft, in particular, a sheet-shaped cell culture, it is preferable that cells are present at a high-density, for example, a density reaching confluence, that is, cells are present at a density that is assumed to cause cells to cover an adhesion surface of the culture container when seeded. The "density reaching confluence" may be typically a density at which cells are expected to contact each other, a density at which contact inhibition occurs, or a density at which proliferation of the cells is substantially stopped by the contact inhibition or higher. The density may be, but is not limited to, for example about 5.0×10⁵ cells/cm² to about 1.0×10⁷ cells/cm², about 5.0×10⁵ cells/cm² to about 5.0×10⁶ cells/cm², about 5.0×10⁵ cells/cm² to about 3.0×10⁶ cells/cm², about 1.0×10⁶ cells/cm² to about 1.0×10⁷ cells/cm², about 1.0×10⁶ cells/cm² to about 5.0×10⁶ cells/cm², about 1.0×10⁶ cells/cm² to about 3.0×10⁶ cells/cm², about 1.5×10⁶ cells/cm² to about 1.0×10⁷ cells/cm², about 1.5×10⁶ cells/cm² to about 5.0×10⁶ cells/cm², about 1.5×10⁶ cells/cm² to about 3.0×10⁶ cells/cm², about 2.0×10⁶ cells/cm² to about 1.0×10⁷ cells/cm², about 2.0×10⁶ cells/cm² to about 5.0×10⁶ cells/cm², or about 2.0×10⁶ cells/cm² to about 3.0×10⁶ cells/cm². In a preferred embodiment, the amount of the cryopreserved cells is about 7.5×10⁵ cells/cm² to about 3.0×10⁶ cells/cm², and in another preferred embodiment, the amount of the cryopreserved cells is about 1.76×10⁶ cells/cm² to about 2.33×10⁶ cells/cm².

In the culture method and the method for producing a graft of the present disclosure, as described above, the cells cryopreserved by the cryopreservation method described in <1> above are typically used. Therefore, the culture method and the method for producing a graft of the present disclosure may include the cryopreservation method described in <1> above, before the step (a) or the step (A) .

In a preferred embodiment, in the culture method and the method for producing a graft of the present disclosure, at least some steps can be performed in a liquid-tight closed container. Typically, the culture method or the method for producing a graft of the present disclosure can be performed by aseptically enclosing the cell population cryopreserved by the method of <1> in the liquid-tight closed container, and thawing the cryopreserved cell population in the liquid-tight closed container. Therefore, in the culture method and the method for producing a graft of the present disclosure, all of the steps are performed in the liquid-tight closed container.

### <3> Graft of Present Disclosure

Another aspect of the present disclosure relates to a graft produced by the production method of the present disclosure. The graft of the present disclosure is useful for a disease to be improved by application of the graft, for example, for treatment of various diseases relating to tissue abnormality. Therefore, in an embodiment, the graft of the present disclosure is used for a disease to be improved by application of the graft, and in particular, for treatment of a disease relating to tissue abnormality. Since the graft of the present disclosure has a higher mechanical strength than the graft according to the related art and has the same inherent properties of constituent cells as those of the graft according to the related art, the graft of the present disclosure can be applied to at least a tissue or a disease that can be treated by the graft containing myoblasts or fibroblasts according to the related art. Examples of the tissue to be treated can include, but are not limited to, myocardium, cornea, retina, esophagus, skin, joint, cartilage, liver, pancreas, gum, kidney, thyroid, skeletal muscles, middle ear, bone marrow, and digestive organs such as stomach, small intestine, duodenum, and large intestine. In addition, examples of the disease to be treated can include, but are not limited to, cardiac diseases (for example, myocardial damage (myocardial infarction or cardiac injury), cardiomyopathy, and the like), corneal diseases (for example, corneal epithelial stem cell deficiency, corneal injury (thermal/chemical corrosion), corneal ulcer, corneal clouding, corneal perforation, corneal cicatrization, Stevens-Johnson syndrome, ocular pemphigoid, and the like), retinal diseases (for example, pigmentary retinopathy, age-related macular degeneration, and the like), esophageal diseases (for example, prevention of inflammation or stenosis of esophagus after esophageal surgery (removal of esophageal cancer), and the like), skin diseases (for example, skin injury (traumatic injury or burn) and the like), joint diseases (for example, degenerative arthritis and the like), cartilage diseases (for example, cartilage injury and the like), liver diseases (for example, chronic hepatopathy and the like), pancreatic diseases (for example, diabetes and the like), dental diseases (for example, periodontal disease and the like), renal diseases (for example, renal insufficiency, renal anemia, renal osteodystrophy, and the like), thyroid diseases (for example, hypothyrosis and the like), muscular diseases (for example, muscle injury, myositis, and the like), middle ear diseases (for example, tympanitis and the like), and bone marrow diseases (for example, leukemia, aplastic anemia, immunodeficiency disease, and the like). Examples of the graft of the present disclosure used for the above diseases are described in Patent Literature 1, Non Patent Literature 1, Tanaka et al., J Gastroenterol. 2013;48(9):1081-9., and the like. The graft of the present disclosure is fragmented to an injectable size and the fragments are injected into a part requiring treatment, such that a higher effect than that of an injection of a single cell suspension can also be obtained (Wang et al., Cardiovasc Res. 2008;77(3):515-24). Therefore, such a utilization method can be used for the graft of the present disclosure.

### <4> Treatment Method of Present Disclosure

Another aspect of the present disclosure relates to a method for treating a disease of a target, the method including applying an effective amount of the graft produced by the method of the present disclosure to the above target requiring the graft. The disease to be treated is the same as described above.

In the present disclosure, the term "treatment" includes all types of medically acceptable preventive and/or therapeutic interventions for the purpose of curing, temporary remission or prevention of a disease, or the like. For example, the term "treatment" includes medically acceptable interventions for various purposes, the interventions including delaying or stopping of progression of a disease associated with tissue abnormality, recession or disappearance of a lesion, prevention of onset of a disease or prevention of recurrence of a disease, and the like.

In the treatment method of the present disclosure, components for enhancing the viability, engraftment properties, and/or functions of the graft and the like, other effective components useful for treatment of a target disease, and the like can be used in combination with such the graft of the present disclosure.

The treatment method of the present disclosure may further include a step of producing a graft of the present disclosure according to the production method of the present disclosure. The treatment method of the present disclosure may further include, before the step of producing the graft, a step of collecting cells for producing a graft (for example, skin cells or blood corpuscles, in the case of using iPS cells) or tissues serving as a supply source of the cells (for example, skin tissues or blood, in the case of using iPS cells) from a subject. In an embodiment, the subject from which the cells or the tissues serving as a supply source of the cells are collected is the same individual as the subject to which a cell culture, a composition, a graft, or the like is to be administered. In another embodiment, the subject from which the cells or the tissues serving as a supply source of the cells are collected is a different individual of the same species as that of the subject to which a cell culture, a composition, a graft, or the like is to be administered. In another embodiment, the subject from which the cells or the tissues serving as a supply source of the cells are collected is an individual of a species different from that of the subject to which a graft, or the like is to be administered.

In the present disclosure, the effective amount is, for example, an amount (for example, size, weight, number of sheets, or the like of the sheet-shaped cell culture) that can suppress the onset or recurrence of a disease, relieve the symptom of a disease, or delay or stop the progression of a disease, and is preferably an amount that prevents the onset or recurrence of the disease or cures the disease. In addition, an amount that does not cause an adverse effect beyond the advantage obtained by the administration is preferable. Such an amount can be appropriately determined, for example, by tests on experimental animals or disease model animals such as mice, rats, dogs, and pigs, and such test methods are well known to those skilled in the art. In addition, the size of the tissue lesion to be treated can be an important index for determination of the effective amount.

Examples of an administration method can include intravenous administration, intramuscular administration, intraosseous administration, intrathecal administration, and direct application to tissues. Although a frequency of administration is typically once per treatment, multiple administrations can also be performed when the desired effect cannot be obtained. When applied to a tissue, the cell culture, composition, sheet-shaped cell culture, or the like of the present disclosure may be immobilized to the target tissue by locking means such as sutures or staples.

### <5> Kit of Present Disclosure

An aspect of the present disclosure relates to a kit for producing a graft by the cryopreservation method and the culture method (the method for producing a graft) of the present disclosure. The kit for producing a graft of the present disclosure includes: a first storage container in which a cell culture substrate and cryopreserved cells frozen on the cell culture substrate are enclosed; and a second storage container in which a cell culture medium is enclosed, the second storage container being connectable to the first storage container in a liquid-tight closed connection manner. Hereinafter, the kit and the use of the kit of the present disclosure will be described with reference to the case where a sheet-shaped cell culture including skeletal myoblasts are produced by way of example.

The kit of the present disclosure includes the first storage container and the second storage container. These containers are configured so that they are connected to each other in a liquid-tight closed connection manner. The "connected to each other in a liquid-tight closed connection manner" means that both the containers are connected to each other as a liquid-tight closed system while maintaining liquid-tight closed properties in each container. Therefore, both the containers can be connected to each other or separated from each other while maintaining an aseptic state.

The cell culture substrate (for example, a culture dish covered with a temperature-responsive material) and the cells (for example, skeletal myoblasts) for transplantation, cryopreserved on the culture substrate are aseptically enclosed in the first storage container. The medium (for example, medium that can be used for sheet-forming incubation, such as DMEM/F12) capable of culturing the cells for transplantation is aseptically enclosed in the second storage container. In a case where the cells for transplantation are cryopreserved, the cells for transplantation may be cryopreserved on the culture substrate and then aseptically enclosed in the first storage container, or the first storage container in which the cells for transplantation placed on the culture substrate (cell culture dish) are aseptically enclosed may be frozen as it is.

When using the kit of the present disclosure, first, the cryopreserved cells are thawed. By doing so, the cell suspension is thawed on the cell culture substrate (cell culture dish). The cell suspension typically contains a cryoprotective agent. Next, the first storage container and the second storage container are connected to each other in a liquid-tight closed connection manner, and the medium aseptically enclosed in the second storage container is added to the cell culture substrate in the first storage container. Accordingly, the cell culture substrate is in a state where a suspension in which the cell population is suspended is contained in the medium containing the cryoprotective agent. Next, the suspension is subjected to graft-forming culture (for example, sheet-forming incubation) for each storage container, thereby obtaining a graft such as a sheet-shaped cell culture. Thus, an aseptic graft can be formed.

As all of the members, methods, or the like for the cryoprotective agent, the medium, and the culture substrate and the like that are used in the kit of the present disclosure, those described above can be used.

### Examples

The present disclosure will be described in more detail with reference to the following examples, but these examples are specific examples of the present disclosure, and the present disclosure is not limited thereto.

### Example 1: Preparation of Sheet-Shaped Cell Culture

Skeletal myoblasts (including fibroblasts) prepared from a human skeletal muscle by a common method were suspended in MCDB131 media containing 600, 300, 200, 100, and 50 µL of 10% DMSO, respectively, so that 2×10⁷ cells were contained in each medium, and the cells were cryopreserved at -150°C. A cell suspension was obtained by thawing the cryopreserved cells in warm water of 37°C, the cell suspension was diluted by adding 10 mL of a 20% human serum-containing DMEM/F12 medium (Thermo Fisher Scientific Inc.), and the diluted medium was seeded to a temperature-sensitive culture dish (UpCell (registered trademark) 3.5 cm, CellSeed Inc.). In this case, 10 µL of the cell suspension was sampled and stained with trypan blue, and the cells were counted. Thereafter, sheet-forming incubation was performed under 37°C and 5% CO₂ for 12 to 26 hours. After the sheet-forming incubation was performed, the medium was removed, 1000 µL of cooled HBSS (+) (Thermo Fisher Scientific Inc.) was added, and then the resultant was left to stand for 10 minutes. Then, a sheet-shaped cell culture was completely detached by gentle pipetting.

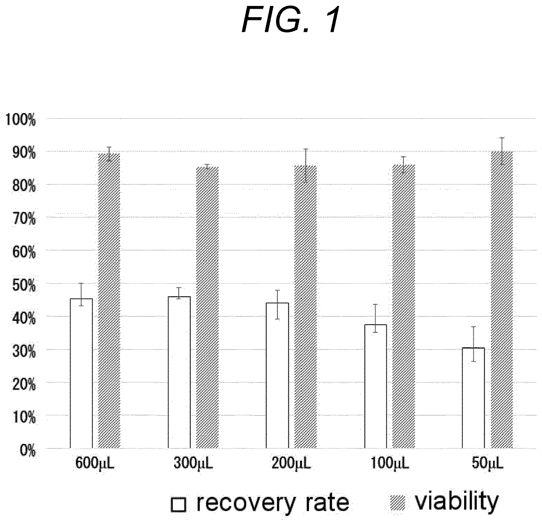

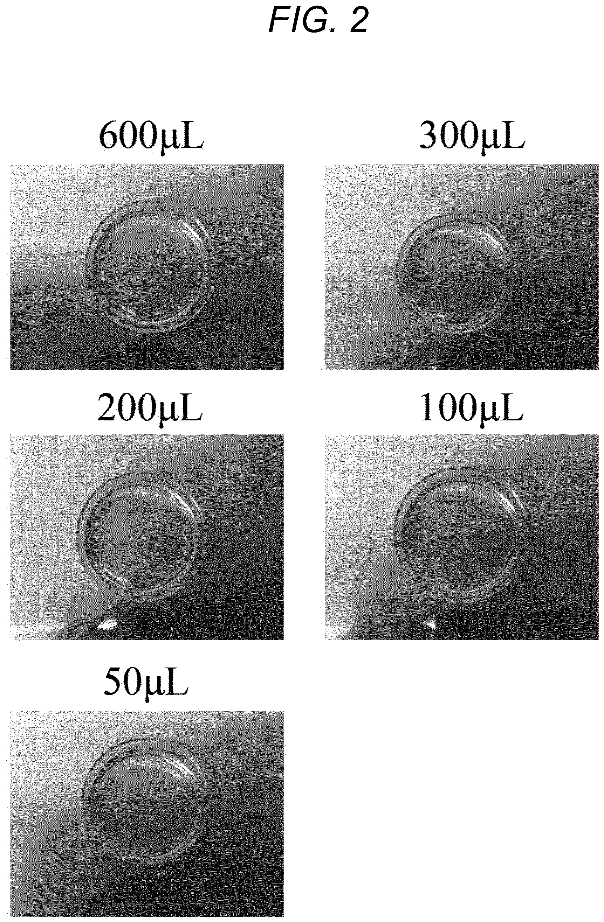

The results are shown in Figs. 1 and 2.

It was observed that when the amount of the cryoprotective agent for the cells was 200 µL or more (that is, density of 1.0×10⁷ cells/mL or less), a recovery rate did not decrease. In addition, it could be appreciated that, because the sheet was formed under all conditions, the sheet formation was performed even though the cryoprotective agent was contained.

### Industrial Applicability

According to the method and the kit of the present disclosure, a graft such as a sheet-shaped cell culture that can be suitable for a medical grade can be efficiently produced. In particular, by using the kit of the present disclosure, the material of the graft can be aseptically transferred and the graft can be aseptically produced even in a medical institution or the like with no a large facility such as a CPC. Therefore, it is expected that the kit contributes to supply of regenerative medicine using a graft.

## Claims

1. A method for culturing cells for transplantation, comprising incubating a cell population in a medium containing a cryoprotective agent.

2. The method according to claim 1, wherein the method includes the following steps of:
(a) obtaining a cell suspension containing a cryoprotective agent by thawing a cell population cryopreserved on a culture substrate;
(b) diluting the cryoprotective agent by adding a culture medium to the cell suspension obtained in (a); and
(c) incubating the cell suspension obtained in (b) on the culture substrate.

3. The method according to claim 1 or 2, wherein the cell population includes adhesion cells.

4. The method according to any one of claims 1 to 3, wherein the cell population includes myoblasts or cardiomyocytes.

5. The method according to any one of claims 1 to 4, wherein the cryoprotective agent is DMSO.

6. The method according to any one of claims 2 to 5, wherein the culture substrate has a cell-adhesive surface of cell culture.

7. The method according to any one of claims 2 to 6, wherein the culture substrate is covered with a temperature-responsive material.

8. The method according to any one of claims 2 to 5, wherein the culture substrate has a culture surface for spheroid formation.

9. A method for producing a graft, comprising the following steps of:
(A) obtaining a cell suspension containing a cryoprotective agent by thawing a cell population including cells for transplantation, cryopreserved on a culture substrate;
(B) diluting the cryoprotective agent by adding a culture medium to the cell suspension obtained in (A); and
(C) performing graft-forming culture on the cell suspension obtained in (B) on the culture substrate.

10. The method according to claim 9, wherein the graft is a sheet-shaped cell culture.

11. The method according to claim 9 or 10, wherein the cells for transplantation are myoblasts or cardiomyocytes.

12. The method according to any one of claims 9 to 11, wherein the cells for transplantation are present in the cell suspension at a density of 7.5×10⁵ cells/cm² to 3.0×10⁶ cells/cm² with respect to an area of the culture substrate.

13. A kit for producing a graft, comprising: a first storage container in which a cell culture substrate and cryopreserved cells frozen on the cell culture substrate are enclosed; and a second storage container in which a cell culture medium is enclosed, the second storage container being connectable to the first storage container in a liquid-tight closed connection manner.
